(19) Europäisches Patentamt
European Patent Office
Office européen des brevets

(11) Publication number: 0 029 934
A1

(12) **EUROPEAN PATENT APPLICATION**

(21) Application number: 80106869.3

(22) Date of filing: 07.11.80

(51) Int. Cl.[3]: C 07 D 491/052
A 61 K 31/505
//(C07D491/052, 239/00, 311/00)

(30) Priority: 15.11.79 US 94407

(43) Date of publication of application: 10.06.81 Bulletin 81/23

(84) Designated Contracting States: AT BE CH DE FR GB IT LI NL SE

(71) Applicant: SCHERING CORPORATION
2000 Galloping Hill Road
Kenilworth, New Jersey 07033(US)

(72) Inventor: Blythin, David John
487 Mountain Avenue
North Caldwell New Jersey 07006(US)

(74) Representative: Antony, Fritz, Dr. et al,
P.O. Box 601 Winkelriedstrasse 35
CH-6002 Lucerne(CH)

(54) **Benzopyrano derivatives, pharmaceutical compositions containing them and processes for preparing the compounds and compositions.**

(57) Novel compounds of the formula

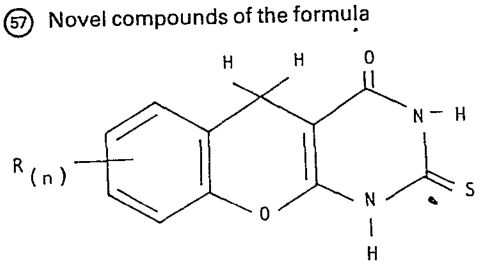

(1)

wherein n is one or two and R represents hydrogen, loweralkyl, lowercycloalkyl, acyloxyloweralkyl, hydroxyloweralkyl, loweralkoxyloweralkyl, nitro, halogeno, hydroxy, loweralkoxy, carboxy, loweralkoxycarbonyl, tetrazolyl, cyano or a carboxamido moiety of the structure

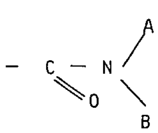

wherein A is straight or branched chain alkyl with up to 12 carbon atoms, lowercycloalkyl, lowercycloalkylloweralkyl, loweralkoxyloweralkyl, hydroxyloweralkyl, fluoroloweralkyl, loweralkenyl, loweralkylthioloweralkyl, loweralkylsulfoxyloweralkyl, loweralkylsulfonylloweralkyl, thiazolyl, oxazolyl, thiadiazolyl, methylthiadiazolyl, furyl, pyrazolyl, tetrazolyl, methyltetrazolyl, phenyl or the grouping $-E-R_8$, wherein E is a straight or branched chain or cyclic loweralkylene optionally substituted by hydroxy, alkanoyloxy, or phenyl, $R_8$ is phenyl, thiazolyl, oxazolyl, thiadiazolyl, methylthiadiazolyl, tetrazolyl, methyltetrazolyl, furyl, pyridiyl, methylpyridyl or piperidinyl; and B is hydrogen, loweralkyl, lowercycloalkyl, lowercycloalkylloweralkyl, or loweralkenyl; or A and B, when taken together with the nitrogen atom to which they are attached, represent imidazolyl, morpholinyl, pyrrolidinyl, piperidinyl or piperazinyl said heterocyclic rings being optionally substituted by hydroxy, loweralkyl or hydroxyloweralkyl; and pharmaceutically acceptable salts thereof.

The preparation of these compounds by four methods is described, in particular one method which comprises reacting a correspondingly R-substituted salicylaldehyde and thiobarbituric acid in an alcoholic solvent in the presence of a sulfonic acid. Representative compounds of the invention have been shown to exhibit anti-allergy reactions and are thus useful in treating such diseases as asthma, allergic rhinitis, urticaria and ulcerative colitis.

EP 0 029 934 A1

BENZOPYRANO DERIVATIVES, PHARMACEUTICAL COMPOSITIONS CONTAINING THEM AND PROCESSES FOR PREPARING THE COMPOUNDS AND COMPOSITIONS.

This invention relates to novel (1H,3H,5H)-(1)-benzopyrano-(2,3-d)-pyrimidine-4-one-2-thiones and the pharmaceutically acceptable salts thereof, to compositions containing them, to the processes for making such compounds and compositions. The compounds are useful as anti-allergy agents in the treatment of such disease states as asthma, allergic rhinitis, urticaria, hay fever and ulcerative colitis.

The novel compounds of this invention are of the formula

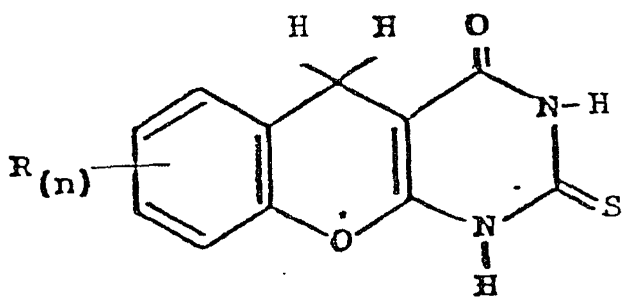

(I)

wherein n is one or two and R represents hydrogen, loweralkyl, lowercycloalkyl, acyloxyloweralkyl, hydroxyloweralkyl, loweralkoxyloweralkyl, nitro, halogeno, hydroxy, loweralkoxy, carboxy, loweralkoxycarbonyl, tetrazolyl, cyano or a carboxamido moiety of the structure

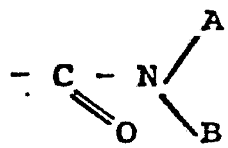

wherein A is straight or branched chain alkyl with up to 12 carbon atoms, lowercycloalkyl, lowercycloalkylloweralkyl, loweralkoxyloweralkyl, hydroxyloweralkyl, fluoroloweralkyl, loweralkenyl, loweralkylthiolower-alkyl, loweralkylsulfoxyloweralkyl, loweralkylsulfonyl-loweralkyl, thiazolyl, oxazolyl, thiadiazolyl, methyl-thiadiazolyl, furyl, pyrazolyl, tetrazolyl, methyl-tetrazolyl, phenyl, or the grouping $-E-R_8$, wherein E is a straight or branched chain or cyclic loweralkylene optionally substituted by hydroxy, alkanoyloxy or phenyl, $R_8$ is phenyl, thiazolyl, oxazolyl, thiadiazolyl, methylthiadiazolyl, tetrazolyl, methyltetrazolyl, furyl, pyridyl, methylpyridyl or piperidinyl; and B is hydrogen, loweralkyl, lowercycloalkyl, lowercycloalkylloweralkyl, or loweralkenyl; or A and B, when taken together with the nitrogen atom to which they are attached, represent imidazolyl, morpholinyl, pyrrolidinyl, piperidinyl or piperazinyl said heterocyclic rings being optionally substituted by hydroxy, loweralkyl or hydroxyloweralkyl; and include the pharmaceutically acceptable salts thereof.

As employed herein, the term "halogeno" refers to fluoro, chloro, bromo and iodo. The term "lower", as it modifies radicals, such as "alkyl", "alkenyl", "alkoxy" or "cycloalkyl", defines those radicals having up to seven carbon atoms. The term "loweralkyl" includes methyl, ethyl, propyl, butyl, pentyl, hexyl and heptyl and isomers thereof, such as isopropyl, t-butyl, neopentyl, 2,3-dimethylbutyl and the like."Lowercycloalkyl" includes cyclopropyl, cyclobutyl, cyclopentyl, cyclohexyl and cycloheptyl. The term "acyloxyloweralkyl" includes those radicals embraced by the partial structure $(-R_4-O-\overset{O}{\overset{\|}{C}}-R_6)$, "loweralkoxyloweralkyl" includes those radicals embraced by the partial structure $(-R_4-O-R_1)$, and "alkanoyloxy" includes those radicals embraced by the partial structure $(-O-\overset{O}{\overset{\|}{C}}-R_1)$, of which acetoxy is preferred, "acyl" includes those radicals embraced by the partial structure $(-\underset{O}{\underset{\|}{C}}-R_6)$.

In the above partial structures $R_4$ is loweralkylene, $R_6$ is a straight or branched chain alkyl radical with up to 12 carbon atoms and $R_1$ is loweralkyl.

In a preferred group of compounds of formula I n is one and the R substituent is located at the 7- or 8-position. At least one substituent in a di-substituted compound is preferably in the 7- or 8-position.

Preferred R-substituents are halogen, carboxy, loweralkoxycarbonyl, acyloxyloweralkyl and the carboxamido moiety, as it is defined above. Within the definition of the carboxamido moiety A is preferably tetrazolyl or the grouping $-E-R_8$, wherein E is straight chain alkylene with up to three carbon atoms and is optionally substituted by hydroxy, and $R_8$ is phenyl, tetrazolyl or pyridyl; and B is hydrogen or loweralkyl, or A and B, when taken together with the nitrogen atom to which they are attached, represent N-imidazolyl; the carboxamido moiety being in position 7.

Particularly preferred compounds are 7-(5-tetrazolylaminocarbonyl)-(1H,3H,5H)-(1)-benzopyrano-(2,3-d)-pyrimidine-4-one-2-thione and 7-(2-[2-pyridyl]ethylaminocarbonyl)-(1H,3H,5H)-(1)-benzopyrano-(2,3-d)-pyrimidine-4-one-2-thione and 7-(N-imidazolylcarbonyl)-(1H,3H,5H)-(1)-benzopyrano-(2,3-d)-pyrimidine-4-one-2-thione.

As the compounds are acidic in character they will form physiologically acceptable (i.e., pharmaceutically acceptable) metal or amine cation salts. Illustrative examples of such metals are the alkali metal, i.e., lithium, sodium and potassium, and the alkaline earth metals, i.e., magnesium and calcium. Other metals, i.e., aluminum, zinc and iron are also within the scope of this invention. Illustrative of the amines are those derived from primary, secondary or tertiary amines. Examples of suitable amines are methylamine, dimethylamine, triethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine and like aliphatic, cycloaliphatic and araliphatic amines containing up to and including about 18 carbon atoms, as well as heterocyclic amines, i.e., piperidine, morpholine, pyrrolidine, piperazine and lower alkyl derivatives thereof, i.e., 1-methyl piperidine, 4-ethylmorpholine, 1-isopropylpyrrolidine, 2-methylpyrrolidine, 1,4-dimethylpiperazine, 2-methylpiperidine, 1,4-dimethylpiperazine, 2-methylpiperidine

and the like, as well as amines containing water solubilizing or hydrophilic groups, i.e., mono-, di-, and triethanolamine, ethyldiethanolamine, N-butylethanolamine, 2-amino-1-butanol, 2-amino-2-ethyl-1,3-propanediol, 2-amino-2-methyl-1-propanol, tris (hydroxymethyl) aminomethane, N-phenylethanolamine, N-(p-tetramylphenyl) diethanolamine, galactamine, N-methyl-D-glucamine, N-methylglucosamine and the like.

The compounds of formula I may be prepared by applying one of the following steps A to D

A) reducing a compound of the formula

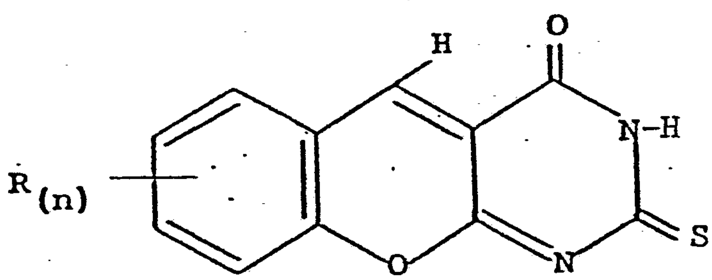

(IV)

wherein R and n are as defined above; or

B) for the preparation of compounds of formula I, wherein R is not acyloxyloweralkyl; cyclising a compound of the formula

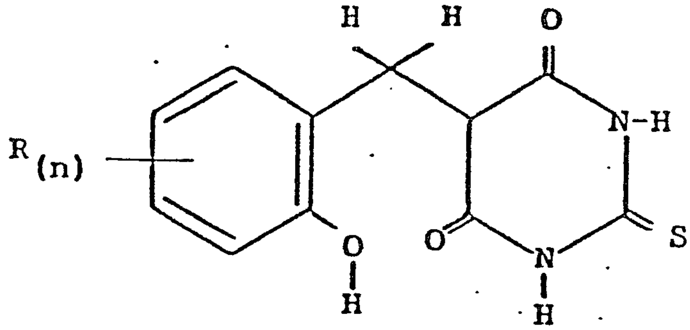

(VII)

wherein R and n are as defined above, with the proviso that R is not acyloxyloweralkyl; and wherein the hydroxy group in position 2 of the phenyl moiety may be protected; or

C) reacting a compound of the formula

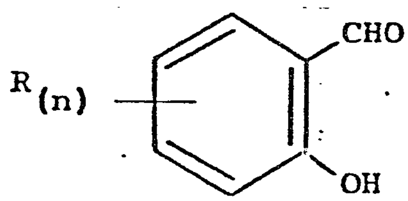

(II)

wherein R and n are as defined above, with 2-thiobarbituric acid in an alcoholic solvent and in the presence of a sulfonic acid; or

D) for the preparation of compounds of formula I, wherein R and n are as defined above, with the same proviso for substituent R as in process step B); reacting a compound of the formula

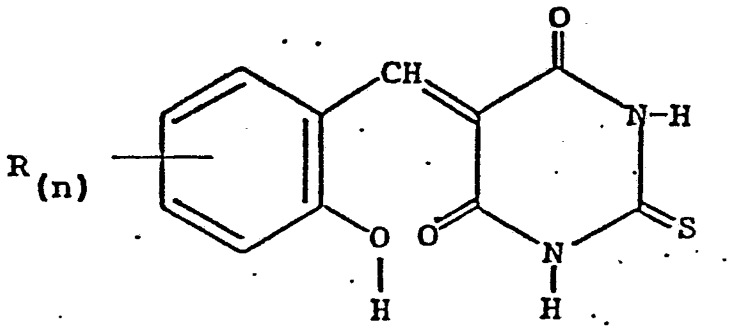

(VI)

in an alcoholic solvent and in the presence of a sulfonic acid;

any of these steps A) to D) being followed by one or more of steps (i) to (xiii) to obtain a compound of formula I or to convert a compound of formula I into another compound of formula I:

(i) esterification of the carboxyl group representing substituent R;

(ii) de-esterification of an alkoxycarbonyl group;

(iii) trans-esterification of an alkoxycarbonyl group;

(iv) acylation of any hydroxy group in substituent R or E;

(v) de-acylation of acyloxyloweralkyl;

(vi) de-etherification of alkoxyloweralkyl;

(vii) etherification of any hydroxyl group in/or representing substituent R;

(viii) hydrolysing or alcoholysing the cyano group representing substituent R;

(ix) oxidation of a hydroxymethyl group representing substituent R;

(x) amidation of the carboxyl group representing substituent R to obtain the carboxamido moiety as defined above;

(xi) trans-amidation of a carboxamido moiety representing substituent R;

(xii) de-amidation of a carboxamido moiety representing substituent R;

(xiii) preparing a pharmaceutically acceptable salt of any of the compounds so obtained.

In step A) reduction of a compound of formula IV is effected by standard reduction techniques, preferably by using excess molar quantities of sodium borohydride in an alcoholic medium. The reduction is preferably effected at room temperature over a period of several days or at least until a yellow-orange coloration is discharged. Excess sodium borohydride is carefully decomposed by the addition of water and acid. If substituent R is susceptible to chemical modifications in the presence of sodium borohydride then neutral conditions such as provided for by sodium cyanoborohydride are advantageously employed.

Alternatively, the desired compound of formula I can also be obtained from a compound of formula IV by heating the latter in the presence of a sulfonic acid, such as p-toluenesulfonic acid or methanesulfonic acid, in an alcoholic solvent, such as propanol or butanol.

The following reaction scheme illustrates the reaction thereby showing the reaction from starting materials well known in the art:

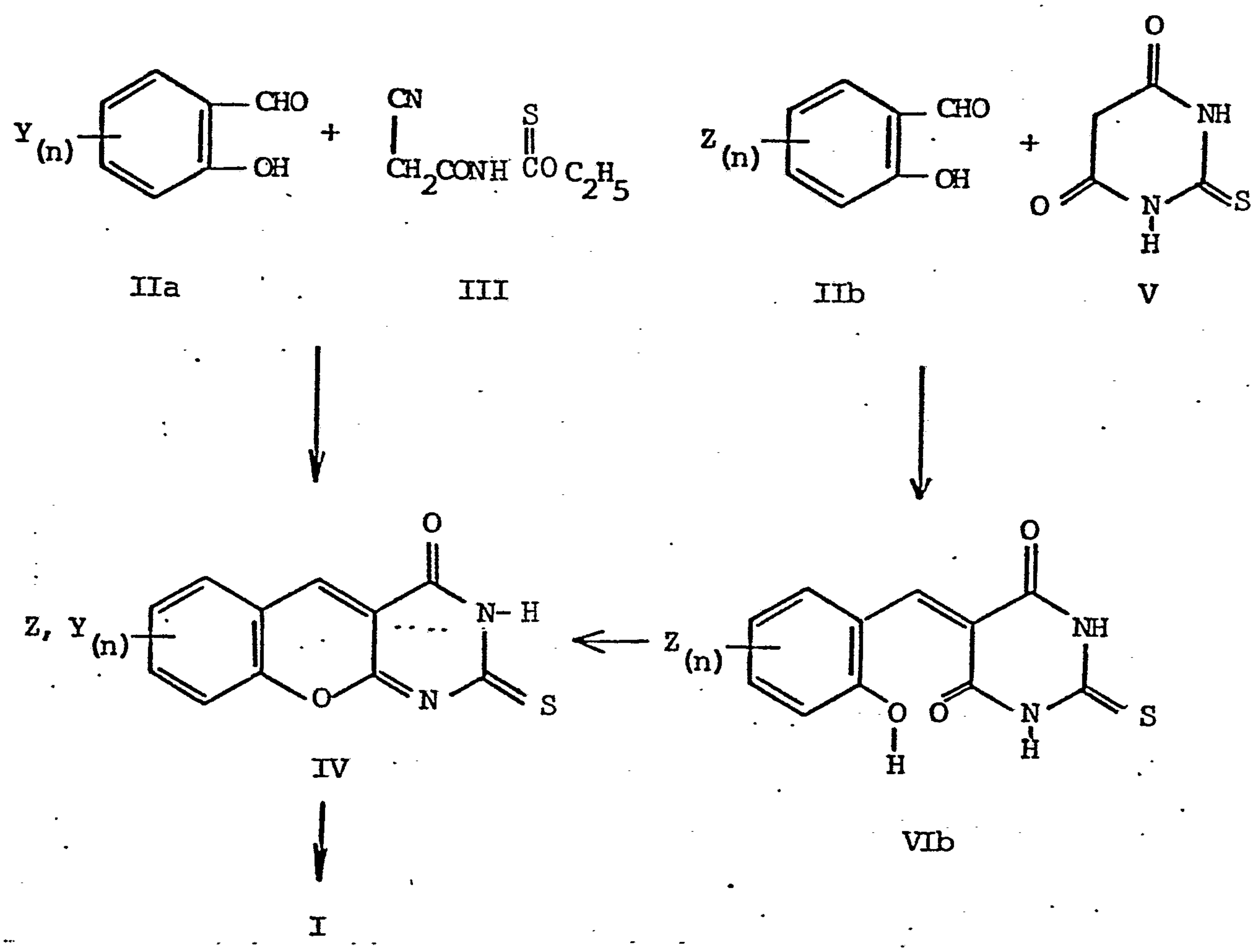

IIa III IIb V

IV VIb

I

wherein n is as previously defined, Y is an electron-donating radical within the scope of R and Z is an electron-withdrawing radical within the scope of R with R being as previously defined. Electron-donating groups within the scope of R are such groups as hydrogen, loweralkyl, lowercycloalkyl, acyloxyloweralkyl, hydroxyloweralkyl, loweralkoxy and loweralkoxyloweralkyl. Electron-withdrawing groups within the scope of R are such groups as loweralkoxycarbonyl, dihalogeno, nitro, carboxy, carboxamido and cyano.

In those instances wherein the initial reaction is to condense the substituted salicylaldehyde (IIa) with the cyanoacetylthiourethane there is produced an appropriately Y-substituted (3H)-(1)-benzopyrano-(2,3-d)-pyrimidine-4-one-2-thione intermediate (IV) which is isolated and may then be converted to a final product (I). The condensation is preferably effected in an alcoholic solvent, preferably ethanol, and catalytic quantities of a secondary base, preferably morpholine although other equivalently functioning bases may be employed. Generally, equimolar quantities of the reactants may be employed although in practice it is preferred to have a slight excess of the cyanoacetylthiourethane. The reaction is preferably effected at reflux temperature and is continued for

times up to eight hours after a yellow-orange precipitate starts to form.

In those instances wherein the initial reaction involves the condensation of an appropriately substituted salicylaldehyde (IIb) with 2-thiobarbituric acid (V), there is produced a 5-[(2-hydroxy)-$Z_{(n)}$-phenylmethylene]-(1H,3H)-pyrimidine-4,6-dione-2-thione (VIb) intermediate. This condensation reaction is effected by heating an admixture of reactants at elevated temperatures (60-100°C), preferably 90°C, in an inert organic solvent (preferably aqueous dioxan) for about 0.5-12 hours. The intermediate (VIb) is then converted into intermediate (IV) by standard dehydration techniques such as employing an acid anhydride, e.g. acetic anhydride, at reflux temperature.

In the above reaction scheme, specifically in compound (IV), the phenyl substituent $R_{(n)}$ is shown as $Z,Y_{(n)}$. As per the definitions of R, Z and Y it is apparent that R and (Z,Y) are synonymous.

In process step B) cyclisation of a compound (VII) is effected by standard dehydration techniques such as by heating the compound (VII) in the presence of polyphosphoric acid or phosphorous oxychloride, or by

heating the compound (VII) with sulfonic acids (preferably methanesulfonic acid) in the presence of such catalysts as phosphorous pentoxide or the like. Alternatively, a mixture of a compound (VII) and a pyridine hydrohalide is heated to melting.

The following reaction scheme illustrates the reaction thereby showing the reaction from starting materials well known in the art or described hereinabove:

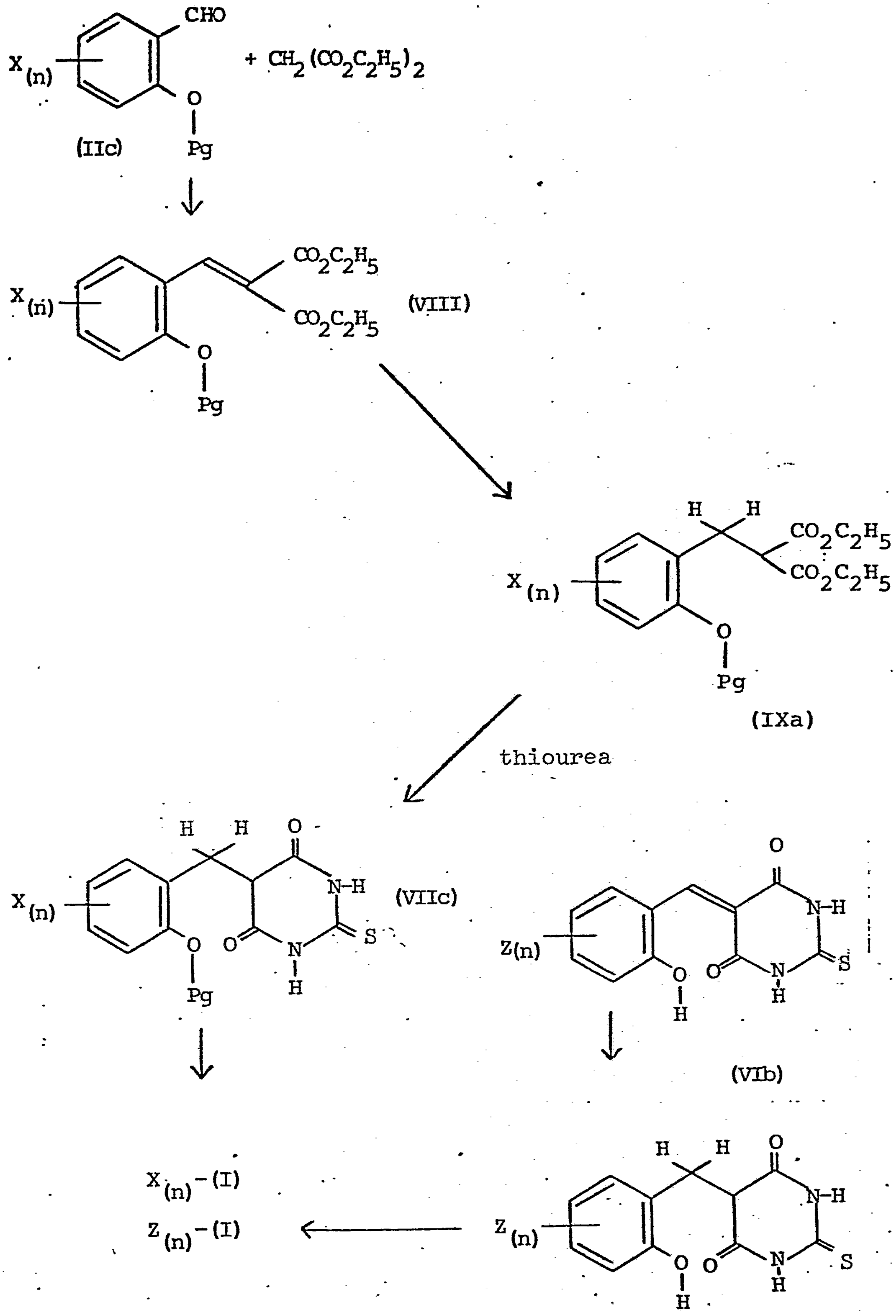


(IIc)

(VIII)

(IXa)

(VIIc)

(VIb)

(VIIb)

$X_{(n)}$-(I)

$Z_{(n)}$-(I)

wherein Z and n are as previously defined, Pg is a hydroxyprotective group, such as benzyl, and X is the same as R being previously defined with the proviso that it is not acyloxyloweralkyl or carboxy.

In the above reaction scheme the substituted salicylaldehyde IIc, the 2-hydroxy group of which is protected, is condensed with diethyl malonate according to methods known in the art to yield the benzylidene malonate (VIII). The compound of formula (VIII) is then reduced to compound (IXa) which thereafter is reacted with thiourea according to a method known in the art (J.A. Vida, et al, J. Med. Chem., 17, 732 (1974)) to yield the pyrimidinedione-thione (VIIc).

Alternatively, a compound of formula (VIb) as described in a previous reaction scheme is reduced to a pyrimidine-dione-thione (VIIb). This reduction as well as the reduction of compound (VIII) is effected according to such known methods as using sodiumborohydride or, if substituent Z is susceptible to chemical modification, sodiumcyanoborohydride providing neutral conditions.

It is readily apparent from the above reaction scheme and the definitions of R,X and Z that R, if it is not acyloxyloweralkyl, corresponds to the scope of

X and Z when taken together. Thus, compounds (VIIb) and (VIIc), if taken together, are also identified herein as compounds (VII) having a R-substituent (with a proviso) rather than an X- or Z-substituent.

The reaction in process step C) comprises the reaction of a substituted salicylaldehyde (II) with barbituric acid in an alcoholic solvent, such as n-propanol, n-butanol or iso-propanol, and in the presence of a sulfonic acid, such as methanesulfonic acid or p-toluenesulfonic acid. Apparently, intermediates are formed in situ during this reaction which have the structure of compounds (VIb) and also the structure of compounds (IV). If these compounds are prepared as described in connection with the two reaction schemes above and are subjected to the reaction conditions of process step C), compounds of formula (I) are also obtained. Accordingly, in process step D) the same reaction conditions ought to be applied as described in relation to process step C). The compounds of formula (VI) may be obtained in a manner shown in the above reaction scheme for compounds (VIb).

All of the facultative finishing steps represent chemical reactions well known in the art such as esterification, amidation, acylation and the like. They are useful when

particular R-substituted compounds must be prepared and the correspondingly substituted starting materials are not available or the use thereof would reduce the overall yield.

The following examples illustrate the invention.

## EXAMPLE I

### 7-CYANO-(1H,3H,5H)-(1)-BENZOPYRANO-(2,3-d)-PYRIMIDINE-4-ONE-2-THIONE

To n-butanol (100 ml.) add 5-cyano-salicylaldehyde (6 g.), 2-thiobarbituric acid (6 g.) and methanesulfonic acid (10 ml.). With stirring, heat the mixture to reflux for 1-1/2 hours. Cool, filter, and wash the solid with isopropanol and ether to yield 7-cyano-(1H,3H,5H)-(1)-benzopyrano-(2,3-d)-pyrimidine-4-one-2-thione, m.p. 320°C (dec).

Similarly, by substituting the 5-cyano-salicylaldehyde of this example with the appropriately R-substituted salicylaldehyde and by following the foregoing procedure, there are produced:

7-nitro-(1H,3H,5H)-(1)-benzopyrano-(2,3-d)-pyrimidine-4-one-2-thione,

7-chloro (1H,3H,5H)-(1)-benzopyrano-(2,3-d)-pyrimidine-4-one-2-thione,

7-bromo-(1H,3H,5H)-(1)-benzopyrano-(2,3-d)pyrimidine-4-one-2-thione,

7-methoxycarbonyl-(1H,3H,5H)-(1)-benzopyrano-(2,3-d)-pyrimidine-4-one-2-thione,

8-ethoxycarbonyl-(1H,3H,5H)-(1)-benzopyrano-(2,3-d)-pyrimidine-4-one-2-thione,

7-nitro-8-trifluoromethyl-(1H,3H,5H)-(1)-benzopyrano-(2,3-d)-pyrimidine-4-one-2-thione,

7-cyano-8-chloro-(1H,3H,5H)-(1)-benzopyrano-(2,3-d)-pyrimidine-4-one-2-thione,

9-methoxy-(1H,3H,5H)-(1)-benzopyrano-(2,3-d)-pyrimidine-4-one-2-thione,

7-tetrazolyl-(1H,3H,5H)-(1)-benzopyrano-(2,3-d)-pyrimidine-4-one-2-thione,

7-methoxy-(1H,3H,5H)-(1)-benzopyrano-(2,3-d)-pyrimidine-4-one-2-thione,

7-methyl-(1H,3H,5H)-(1)-benzopyrano-(2,3-d)-pyrimidine-4-one-2-thione,

7,8-dimethyl-(1H,3H,5H)-(1)-benzopyrano-(2,3-d)-pyrimidine-4-one-2-thione,

(1H,3H,5H)-(1)-benzopyrano-(2,3-d)-pyrimidine-4-one-2-thione, m.p. 295-299°C (dec.)

## EXAMPLE II

### 7-CARBOXY-(1H,3H,5H)-(1)-BENZOPYRANO-(2,3-d)-PYRIMIDINE-4-ONE-2-THIONE

Warm a suspension of 7-methoxycarbonyl-(1H,3H,5H)-(1)-benzopyrano-(2,3-d)-pyrimidine-4-one-2-thione (5 g.) in 1N-sodium hydroxide (25 ml.) until a clear solution is

formed and continue the warming for an additional 1/2 hour. Filter the solution and acidify the filtrate with 2N sulfuric acid. Filter and wash the solids with water, isopropanol and then ether. Dry the washed solid to obtain

7-carboxy-(1H,3H,5H)-(1)-benzopyrano-(2,3-d)-pyrimidine-4-one-2-thione, m.p. 295°C (dec.)

In a similar manner, by the use of the appropriately substituted starting material and by following substantially the process of this example there is produced:

6-carboxy-(1H,3H,5H)-(1)-benzopyrano-(2,3-d)-pyrimidine-4-one-2-thione,

8-carboxy-(1H,3H,5H)-(1)-benzopyrano-(2,3-d)-pyrimidine-4-one-2-thione,

9-carboxy-(1H,3H,5H)-(1)-benzopyrano-(2,3-d)-pyrimidine-4-one-2-thione,

7,8-dicarboxy-(1H,3H,5H)-(1)-benzopyrano-(2,3-d)-pyrimidine-4-one-2-thione.

## EXAMPLE III

## 7-(IMIDAZOLYLCARBONYL)-(1H,3H,5H)-(1)-BENZOPYRANO-(2,3-d)-PYRIMIDINE-4-ONE-2-THIONE

Add N,N'-carbonyldiimidazole (70 g.) in portions to a suspension of 7-carboxy-(1H,3H,5H)-(1)-benzopyrano-(2,3-d)-pyrimidine-4-one-2-thione (30 g.) in dry dimethylformamide (300ml.). Heat to 70°C for 3 hours. Cool, filter, wash with ether and dry to give 7-(imidazolylcarbonyl)-(1H,3H,5H)-(1)-benzopyrano-(2,3-d)-pyrimidine-4-one-2-thione, m.p. 226-227°C.

In a similar manner, by the use of the appropriately carboxyl-substituted compounds (as prepared by the technics of Example II), and by following substantially the process of this example there is produced the corresponding imidazolylcarbonyl substituted (or disubstituted) (1H,3H,5H)-(1)-benzopyrano-(2,3-d)-pyrimidine-4-one-2-thione.

## EXAMPLE IV

## 7-(5-TETRAZOLYLAMINOCARBONYL)-(1H,3H,5H)-(1)-BENZOPYRANO-(2,3-d)-PYRIMIDINE-4-ONE-2-THIONE

Suspend 7-(imidazolylcarbonyl)-(1H,3H,5H)-(1)-benzopyrano-(2,3-d)-pyrimidine-4-one-2-thione (0.6g.) in dry dimethylformamide (20ml.). With stirring, add 5 aminotetrazole (0.61 g.) and warm the mixture to 65°-75°C and keep at that temperature for about 3/4 hour. Allow the mixture to cool, pour into water and acidify with hydrochloric acid. Filter and wash with isopropanol and ether

to yield 7-(5-tetrazolylaminocarbonyl)-(1H,3H,5H)-(1)-benzopyrano-(2,3-d)-pyrimidine-4-one-2-thione, m.p.>350°C.

Prepare in essentially the same way except that if a basic ring is present, e.g., pyridine, the basic reaction product is either neutralized with acid, or acidified with HCl and the product isolated as the hydrochloride salt:

7-methylaminocarbonyl-(1H,3H,5H)-(1)-benzopyrano-(2,3-d)-pyrimidine-4-one-2-thione,

7-ethylaminocarbonyl-(1H,3H,5H)-(1)-benzopyrano-(2,3-d)-pyrimidine-4-one-2-thione,

7-n-propylaminocarbonyl-(1H,3H,5H)-(1)-benzopyrano-(2,3-d)-pyrimidine-4-one-2-thione,

7-iso-propylaminocarbonyl-(1H,3H,5H)-(1)-benzopyrano-(2,3-d)-pyrimidine-4-one-2-thione,

7-n-butylaminocarbonyl-(1H,3H,5H)-(1)-benzopyrano-(2,3-d)-pyrimidine-4-one-2-thione,

7-iso-butylaminocarbonyl-(1H,3H,5H)-(1)-benzopyrano-(2,3-d)-pyrimidine-4-one-2-thione,

7-sec-butylaminocarbonyl-(1H,3H,5H)-(1)-benzopyrano-(2,3-d)-pyrimidine-4-one-2-thione,

7-tert-butylaminocarbonyl-(1H,3H,5H)-(1)-benzopyrano-(2,3-d)-pyrimidine-4-one-2-thione,

7-n-pentylaminocarbonyl-(1H,3H,5H)-(1)-benzopyrano-(2,3-d)-pyrimidine-4-one-2-thione,

7-n-hexylaminocarbonyl-(1H,3H,5H)-(1)-benzonpyrano-(2,3-d)-pyrimidine-4-one-2-thione,

7-n-octylaminocarbonyl-(1H,3H,5H)-(1)-benzopyrano-(2,3-d)-pyrimidine-4-one-2-thione,

7-n-decylaminocarbonyl-(1H,3H,5H)-(1)-benzopyrano-(2,3-d)-pyrimidine-4-one-2-thione,

7-benzylaminocarbonyl-(1H,3H,5H)-(1)-benzopyrano-(2,3-d)-pyrimidine-4-one-2-thione,

7-cyclopropylaminocarbonyl-(1H,3H,5H)-(1)-benzopyrano-(2,3-d)-pyrimidine-4-one-2-thione,

7-cyclobutylaminocarbonyl-(1H,3H,5H)-(1)-benzopyrano-(2,3-d)-pyrimidine-4-one-2-thione,

7-cyclopentylaminocarbonyl-(1H,3H,5H)-(1)-benzopyrano-(2,3-d)-pyrimidine-4-one-2-thione,

7-cyclohexylaminocarbonyl-(1H,3H,5H)-(1)-benzopyrano-(2,3-d)-pyrimidine-4-one-2-thione,

7-cycloheptylaminocarbonyl-(1H,3H,5H)-(1)-benzopyrano-(2,3-d)-pyrimidine-4-one-2-thione,

7-cyclopropylmethylaminocarbonyl-(1H,3H,5H)-(1)-benzopyrano-(2,3-d)-pyrimidine-4-one-2-thione,

7-cyclobutylmethylaminocarbonyl-(1H,3H,5H)-(1)-benzopyrano-(2,3-d)-pyrimidine-4-one-2-thione,

8-methylaminocarbonyl-(1H,3H,5H)-(1)-benzopyrano-(2,3-d)-pyrimidine-4-one-2-thione,

8-ethylaminocarbonyl-(1H,3H,5H)-(1)-benzopyrano-(2,3-d)-pyrimidine-4-one-2-thione,

8-n-propylaminocarbonyl-(1H,3H,5H)-(1)-benzopyrano-(2,3-d)-pyrimidine-4-one-2-thione,

8-iso-propylaminocarbonyl-(1H,3H,5H)-(1)-benzopyrano-(2,3-d)-pyrimidine-4-one-2-thione,

8-n-butylaminocarbonyl-(1H,3H,5H)-(1)-benzopyrano-(2,3-d)-pyrimidine-4-one-2-thione,

8-iso-butylaminocarbonyl-(1H,3H,5H)-(1)-benzopyrano-(2,3-d)-pyrimidine-4-one-2-thione,

8-n-pentylaminocarbonyl-(1H,3H,5H)-(1)-benzopyrano-(2,3-d)-pyrimidine-4-one-2-thione,

7-(2-(2-pyridyl) ethylaminocarbonyl)-(1H,3H,5H)-(1)-benzopyrano-(2,3-d)-pyrimidine-4-one-2-thione,m.p.285-288°C,

7-(2-(3-pyridyl) ethylaminocarbonyl)-(1H,3H,5H)-(1)-benzopyrano-(2,3-d)-pyrimidine-4-one-2-thione,

7-(2- (4-pyridyl) ethylaminocarbonyl)-(1H,3H,5H)-(1)-benzopyrano-(2,3-d)-pyrimidine-4-one-2-thione,

7-(2-pyridylmethylaminocarbonyl) -(1H,3H,5H)-(1)-benzopyrano-(2,3-d)-pyrimidine-4-one-2-thione,

7-(3-pyridylmethylaminocarbonyl)-(1H,3H,5H)-(1)-benzopyrano-(2,3-d)-pyrimidine-4-one-2-thione,

7-(4-pyridylmethylaminocarbonyl)-(1H,3H,5H)-(1)-benzopyrano-(2,3-d)-pyrimidine-4-one-2-thione,

7-(3- (2-pyridyl) propylaminocarbonyl)-(1H,3H,5H)-(1)-benzopyrano-(2,3-d)-pyrimidine-4-one-2-thione,

7-(3- (3-pyridyl) propylaminocarbonyl)-(1H,3H,5H)-(1)-benzopyrano-(2,3-d)-pyrimidine-4-one-2-thione,

7-(3- (4-pyridyl) propylaminocarbonyl)-(1H,3H,5H)-(1)-benzopyrano-(2,3-d)-pyrimidine-4-one-2-thione,

7-(2- (6'-methyl-2'-pyridyl) ethylaminocarbonyl)-(1H,3H, 5H)-(1)-benzopyrano-(2,3-d)-pyrimidine-4-one-2-thione,

8-(2- (2-pyridyl) ethylaminocarbonyl)-(1H,3H,5H)-(1)-benzopyrano-(2,3-d)-pyrimidine-4-one-2-thione,

8-(2- (3-pyridyl) ethylaminocarbonyl)-(1H,3H,5H)-(1)-benzopyrano-(2,3-d)-pyrimidine-4-one-2-thione,

8-(2- (4-pyridyl) ethylaminocarbonyl)-(1H,3H,5H)-(1)-benzopyrano-(2,3-d)-pyrimidine-4-one-2-thione,

8-(2-pyridylmethylaminocarbonyl)-(1H,3H,5H-(1)-benzopyrano-(2,3-d)-pyrimidine-4-one-2-thione,

8-(3- (2-pyridyl) propylaminocarbonyl)-(1H,3H,5H)-(1)-benzopyrano-(2,3-d)-pyrimidine-4-one-2-thione,

8-(2- (6'-methyl-2'-pyridyl) ethylaminocarbonyl)—(1H,3H, 5H)-(1)-benzopyrano-(2,3-d)-pyrimidine-4-one-2-thione,

7-((2'-hydroxy-2'-phenyl)-ethylaminocarbonyl)-(1H,3H,5H)-(1)-benzopyrano-(2,3-d)-pyrimidine-4-one-2-thione,
m.p. 294°C,

8-((2'-hydroxy-2'phenyl)-ethylaminocarbonyl)-(1H,3H,5H)-(1)-benzopyrano-(2,3-d)-pyrimidine-4-one-2-thione,

7-((2'-isobutyryloxy-2'-phenyl)-ethylaminocarbonyl)-(1H,3H, 5H)-(1)-benzopyrano-(2,3-d)-pyrimidine-4-one-2-thione,
m.p. 252-254°C,

7-((2'-acetoxy-2'-phenyl)-ethylaminocarbonyl)-(1H,3H,5H)-(1)-benzopyrano-(2,3-d)-pyrimidine-4-one-2-thione,
m.p. 275-278°C,

7-methoxyethylaminocarbonyl-(1H,3H,5H)-(1)-benzopyrano-(2,3-d)-pyrimidine-4-one-2-thione,

7-hydroxyethylaminocarbonyl-(1H,3H,5H)-(1)-benzopyrano-(2,3-d)-pyrimidine-4-one-2-thione,

7-(3'-hydroxypropylaminocarbonyl)-(1H,3H,5H)-(1)-benzopyrano-(2,3-d)-pyrimidine-4-one-2-thione,

7-dimethylaminocarbonyl-(1H,3H,5H)-(1)-benzopyrano-(2,3-d)-pyrimidine-4-one-2-thione,

7-diethylaminocarbonyl-(1H,3H,5H)-(1)-benzopyrano-(2,3-d)-pyrimidine-4-one-2-thione,

7-morpholinocarbonyl-(1H,3H,5H)-(1)-benzopyrano-(2,3-d)-pyrimidine-4-one-2-thione,

7-pyrrolidinocarbonyl-(1H,3H,5H)-(1)-benzopyrano-(2,3-d)-pyrimidine-4-one-2-thione,

7-piperidinocarbonyl-(1H,3H,5H)-(1)-benzopyrano-(2,3-d)-pyrimidine-4-one-2-thione,

7-(N'-methylpiperazinylcarbonyl)-(1H,3H,5H)-(1)-benzopyrano-(2,3-d)-pyrimidine-4-one-2-thione,

7-(N'-hydroxyethylpiperazinylcarbonyl)-(1H,3H,5H)-(1)-benzopyrano-(2,3-d)-pyrimidine-4-one-2-thione,

8-methoxyethylaminocarbonyl-(1H,3H,5H)-(1)-benzopyrano-(2,3-d)-pyrimidine-4-one-2-thione,

8-hydroxyethylaminocarbonyl-(1H,3H,5H)-(1)-benzopyrano-(2,3-d)-pyrimidine-4-one-2-thione,

8-diethylaminocarbonyl-(1H,3H,5H)-(1)-benzopyrano-(2,3-d)-pyrimidine-4-one-2-thione,

8-morpholinocarbonyl-(1H,3H,5H)-(1)-benzopyrano-(2,3-d)-

pyrimidine-4-one-2-thione,

7-(2'-thiazolylaminocarbonyl)-(1H,3H,5H)-(1)-benzopyrano-(2,3-d)-pyrimidine-4-one-2-thione,

7-(2'-oxazolylaminocarbonyl)-(1H,3H,5H)-(1)-benzopyrano-(2,3-d)-pyrimidine-4-one-2-thione,

7-(3'-pyrazolylaminocarbonyl)-(1H,3H,5H)-(1)-benzopyrano-(2,3-d)-pyrimidine-4-one-2-thione,

7-(2'-methyl-5'-tetrazolylaminocarbonyl)-(1H,3H,5H)-(1)-benzopyrano-(2,3-d)-pyrimidine-4-one-2-thione.

## EXAMPLE V

### 7-(n-BUTYLAMINOCARBONYL)-(1H,3H,5H)-(1)-BENZOPYRANO-(2,3-d)-PYRIMIDINE-4-ONE-2-THIONE

7-(Imidazolylcarbonyl)-(1H,3H,5H)-(1)-benzopyrano-(2,3-d)-pyrimidine-4-one-2-thione (1 g.) is suspended in dry dimethylformamide (60 ml.) and n-butylamine (1.1 g.) is added. The mixture is stirred at room temperature overnight then at 70°C for 1 1/2 hours. The product is added to dilute HCl and the solid is collected, washed with water, ethanol and ether and dried to yield the desired 7-(n-butylaminocarbonyl)-(1H,3H,5H)-(1)-benzopyrano-(2,3-d)-pyrimidine-4-one-2-thione.

## EXAMPLE VI

### 7-(n-PROPYLOXYCARBONYL)-(1H,3H,5H)-(1)-BENZOPYRANO-(2,3-d)-PYRIMIDINE-4-ONE-2-THIONE

To a solution of 5-carboxysalicylaldehyde (6.5 g.)

and 2-thiobarbituric acid (6 g.) in n-propanol (100 ml.), add methanesulfonic acid (10 ml.). Warm the solution gradually, with stirring, to reflux during about 2 hours, then reflux for 1-1/2 hours. Cool, filter and wash the solid with cold ethanol and ether to yield 7-(n-propyloxycarbonyl)-(1H,3H,5H)-(1)-benzopyrano-(2,3-d)-pyrimidine-4-one-2-thione.

Similarly, by substituting the n-propanol of this example with other primary alcohols and heating at 100°-140°C for 1/2-6 hours under pressure, if necessary, there are produced:

7-(n-butyloxycarbonyl)-(1H,3H,5H)-(1)-benzopyrano-(2,3-d)-pyrimidine-4-one-2-thione,

7-(n-pentyloxycarbonyl)-(1H,3H,5H)-(1)-benzopyrano-(2,3-d)-pyrimidine-4-one-2-thione,

7-(2'-methylbutyloxycarbonyl)-(1H,3H,5H)-(1)-benzopyrano-(2,3-d)-pyrimidine-4-one-2-thione,

7-(cyclopentylmethyloxycarbonyl)-(1H,3H,5H)-(1)-benzopyrano-(2,3-d)-pyrimidine-4-one-2-thione.

7-(isopropyloxycarbonyl)-(1H,3H,5H)-(1)-benzopyrano-(2,3-d)-pyrimidine-4-one-2-thione.

7-(isobutyloxycarbonyl)-(1H,3H,5H)-(1)-benzopyrano-(2,3-d)-pyrimidine-4-one-2-thione.

## EXAMPLE VII

## 7-n-BUTYLOXYMETHYL-(1H,3H,5H)-(1)-BENZOPYRANO-(2,3-d)-PYRIMIDINE-4-ONE-2-THIONE

To a solution of 5-hydroxymethyl-salicylaldehyde (5.8 g.) and 2-thiobarbituric acid (6 g.) in n-butanol (100 ml.) add methanesulfonic acid (10 ml.) and heat to reflux, with stirring, for 1 hour. Cool, filter and wash the solid with ethanol and ether to yield 7-n-butyloxymethyl-(1H,3H,5H)-(1)-benzopyrano-(2,3-d)-pyrimidine-4-one-2-thione.

Similarly, by substituting the 5-hydroxymethyl-salicylaldehyde of this example with the appropriately R-substituted salicylaldehyde and the n-butanol with another primary or secondary alcohol having a boiling point of at least 100°C and heating at 100°C-140°C there are produced:

7-n-propyloxymethyl-(1H,3H,5H)-(1)-benzopyrano-(2,3-d)-pyrimidine-4-one-2-thione,

7-n-butyloxyethyl-(1H,3H,5H)-(1)-benzopyrano-(2,3-d)-pyrimidine-4-one-2-thione,

7-n-pentyloxymethyl-(1H,3H,5H)-(1)-benzopyrano-(2,3-d)-pyrimidine-4-one-2-thione.

## EXAMPLE VIII

## 7-HYDROXYMETHYL-(1H,3H,5H)-(1)-BENZOPYRANO-(2,3-d)-PYRIMIDINE-4-ONE-2-THIONE

Reflux a solution of 7-(n-butyloxymethyl)-(1H,3H,5H)-(1)-benzopyrano-(2,3-d)-pyrimidine-4-one-2-thione (5g.) with 48% HBr (100 ml.) for 4 hours. Cool and filter off

0029934

the solid. Dissolve the solid in IN-NaOH (100 ml.) under nitrogen and stir at ambient temperature for 24 hours. Acidify with 5N-HCl, filter, and wash the product with water, ethanol and ether to yield 7-hydroxymethyl-(1H,3H,5H)-(1)-benzopyrano-(2,3-d)-pyrimidine-4-one-2-thione.

Similarly, by following the above procedure there are produced:

7-(2'-hydroxyethyl)-(1H,3H,5H)-(1)-benzopyrano-(2,3-d)-pyrimidine-4-one-2-thione,

8-hydroxymethyl-(1H,3H,5H)-(1)-benzopyrano-(2,3-d)-pyrimidine-4-one-2-thione,

8-(2'-hydroxyethyl)-(1H,3H,5H)-(1)-benzopyrano-(2,3-d)-pyrimidine-4-one-2-thione,

7-(2'-hydroxypropyl)-(1H,3H,5H)-(1)-benzopyrano-(2,3-d)-pyrimidine-4-one-2-thione,

7-hydroxymethyl-9-nitro-(1H,3H,5H)-(1)-benzopyrano-(2,3-d)-pyrimidine-4-one-2-thione,

7-hydroxymethyl-9-chloro-(1H,3H,5H)-(1)-benzopyrano-(2,3-d)-pyrimidine-4-one-2-thione,

## EXAMPLE IX

### 7-(ISOBUTYRYLOXYMETHYL)-(1H,3H,5H)-(1)-BENZOPYRANO-(2,3-d)-PYRIMIDINE-4-ONE-2-THIONE

To a cooled (below 5°C) suspension of 7-hydroxymethyl-(1H,3H,5H)-(1)-benzopyrano-(2,3-d)-pyrimidine-4-one-2-thione (2.8 g.) in dry pyridine (100 ml.), add

isobutyric anhydride (20 ml.). Keep the mixture cold for 24 hours and stir the mixture at 20°C for an additional two days. Pour the mixture over ice water, filter and successively wash the solids with water, ethanol and ether. Dry the washed solids to obtain 7-(isobutyryl-oxymethyl)-(1H,3H,5H)-(1)-benzopyrano-(2,3-d)-pyrimidine-4-one-2-thione.

Similarly, by starting with other pyrimidine-4-one-2-thiones of this invention which bear one or more reactive hydroxyl groups (i.e., when R represents hydroxyalkyl), the foregoing esterification may be affected. Analogously, by utilizing the acid chloride or anhydride of the appropriate acid and following the techniques of this example, the following esters may be prepared: acetyl, propionyl, n-butyryl, isobutyryl, pivaloyl, t-butylacetyl, cyclopropylcarboxyl, cyclobutylcarboxyl, cyclopentylcarboxyl, 1-adamantylcarboxyl, lauroyl, benzoyl and ethyl oxalyl.

## EXAMPLE X

### 7-ETHOXYCARBONYL-(1H,3H,5H)-(1)-BENZOPYRANO-(2,3-d)-PYRIMIDINE-4-ONE-2-THIONE

Suspend 7-carboxy-(1H,3H,5H)-(1)-benzopyrano-(2,3-d)-pyrimidine-4-one-2-thione (5 g.) in ethanol and pass in HCl gas until saturated. Reflux the mixture for 24 hours then cool, filter and wash with ethanol and ether to yield 7-ethoxycarbonyl-(1H,3H,5H)-(1)-benzopyrano-(2,3-d)-pyrimidine-4-one-2-thione.

Similarly can be prepared:

7-(iso-butyloxycarbonyl)-(1H,3H,5H)-(1)-benzopyrano-(2,3-d)-pyrimidine-4-one-2-thione.

## EXAMPLE XI

## ISOLATION OF THE (N-METHYL-D-GLUCAMINE) SALT OF 7-(5'-TETRAZOLYLAMINOCARBONYL)-(1H,3H,5H)-(1)-BENZOPYRANO-(2,3-d)-PYRIMIDINE-4-ONE-2-THIONE

Dissolve N-methyl-D-glucamine (11.4 g.) in water (200 ml.). Add 7-(5'-tetrazolylaminocarbonyl)-(1H,3H,5H)-(1)-benzopyrano-(2,3-d)-pyrimidine-4-one-2-thione (10 g.) and stir until dissolved. Filter and lyophilize to yield the bis-NMG salt of 7-(5'-tetrazolylaminocarbonyl)-(1H,3H,5H)-(1)-benzopyrano-(2,3-d)-pyrimidine-4-one-2-thione, melting behaviour: softens at ca. 120°C, shrivels to opaque gum ca. 128°C.

Similarly may be prepared the salts of any of the compounds disclosed here with the exception that if the compound is a monobasic acid only one equivalent of N-methyl-D-glucamine is used.

The compounds of the invention (I) are anti-allergic agents useful in the prophylactic treatment of sensitized animals for allergy and anaphylactic reaction of a reagin or non-reagin mediated nature and as such are useful in the treatment of such disease states as all forms of asthma, allergic rhinitis, and any allergic condition induced by reaginic antibodies.

In testing the compounds of this invention, the two primary assays utilized are the Passive Cutaneous Anaphylaxis (PCA) assay and the Antigen induced histamine release from rat peritoneal mast cells passively sensitized in vivo assay. The compounds are additionally tested as antagonists of intradermally injected histamine and serotonin; these secondary tests show that the compounds are not working as classical antihistamine or antiserotonin anti-allergy agents.

These tests may be briefly described as follows: Preparation of Antisera Containing Homocytotropic Antibody (IgE) to N. Brasiliensis: Male Sprague-Dawley rats, 150-200 g., were injected subcutaneously with 3000 larvae and 28 days later were reinfected with 3000 larvae. Serum was collected 7-12 days following reinfection and frozen in small aliquots at -40°C.

Preparation of Antigen: Adult worms were harvested from the small intestine of rats 8-11 days after infection with larvae. A homogenate was prepared from a suspension of worms in 0.15 M saline using a ground glass homogenizer. The homogenate was centrifuged at 3000 x g for 15 minutes in a Sorvall RC2-B centrifuge. The supernatant was carefully removed and frozen at -40°C. Just prior to use as antigen, the supernatant was adjusted to a concentration

of 5 mg protein per milliliter. All antigen preparations were standardized in this way although it is recognized that the protein concentration of this crude worm extract does not necessarily reflect the amount of specific antigen present in the extract.

Passive Cutaneous Anaphylaxis (PCA): Male Sprague-Dawley rats weighing 250-300 g were used for PCA reactions. Nine two-fold dilutions of antisera containing homocytotropic antibodies against N. brasiliensis were made in 0.15 M saline. Each dilution was injected intradermally at separate sites onto the shaved backs of normal rats and 48 hours later the animals were challenged intravenously with 0.1 ml antigen (worm extract) mixed with 0.9 ml of one percent Evans blue dye. The animals were sacrificed 45 minutes following antigen challenge, skinned, and the area of blueing measured with a millimeter rule. The diameter of the sites of reaction were graded as follows:

| Diamter | Score |
|---|---|
| 20 (or greater) | 4 |
| 15-19 | 3 |
| 14-10 | 2 |
| 5-9 | 1 |

The intensity of the reaction was also graded from 0-4.

Histamine Release from Rat Peritoneal Mast Cells Passively Sensitized in vivo: The method used in these studies is a modification of that of Orange, et al. Briefly, male Sprague-Dawley rats (CD strain), 150-200 g were injected i.p. with 2.0 ml of a dilution of rat antisera containing HA. Two hours later the animals were challenged with 100 mg worm protein in 5.0 ml Tyrode's solution containing 50 mg/ml of heparin. Exactly five minutes later, the peritoneal fluid was harvested and centrifuged at 150 x g for five minutes at 4°C. The supernatants were removed, and the cells were resuspended in 1.0 ml Tyrode's and boiled for seven minutes to extract residual cell histamine. The supernatant and cell extracts were frozen at -70°C and later assayed for their histamine content.

Histamine Release in vitro from Passively Sensitized Mast Cells: Peritoneal mast cells were obtained from normal animals, pooled, washed and resuspended in Tyrode's minus gelatin buffer. An equal volume of serum containing rat homocytotropic antibody was added to the cell suspension and the mixture was incubated at 37°C for two hours in a metabolic shaker. The suspension was then centrifuged at 150 x g for 10 minutes and the supernatant

discarded. The cells were resuspended in Tyrode's minus gelatin, combined with antigen, and incubated for five minutes at 37°C. The cells were harvested by centrifugation and the supernatants immediately frozen at -70°C. Residual histamine was extracted from the cells by boiling them for seven minutes in 1.0 ml Tyrode's minus gelatin.

Histamine Release in vitro from Actively Sensitized Mast Cells: Peritoneal mast cells were obtained from rats 21-28 days following infection with 3000 Nippostrongylus (NB) larvae. The cells were washed and suspended in Tyrode's buffer. Inhibitor dissolved in Tyrode's was added one minute before antigen. An equal volume of antigen, prewarmed to 37°C, was added and the mixture incubated at 37°C for 15 minutes. The cells and supernatant were then processed as described above for passive sensitization. Both in vitro methods are essentially those described by Wilson, et al.

Histamine Assay: The fluorescent assay of Shore, et al as modified by Technicon for automated determination of histamine was employed.

Determination of Antihistamine and Antiserotonin Activities of Compounds: Normal rats, 200-250 g, were injected i.d. with 10, 20 and 50 mg of histamine and/or

serotonin at separate sites onto their backs 30 or 60 minutes following i.p. or oral administration of drug, respectively. Immediately following the last i.d. injection, the animals received 1 ml. of a one percent solution of Evans blue dye intravenously. Fifteen minutes later they were sacrificed, and the area of blueing measured with a millimeter rule. Each compound was tested for antihistamine or antiserotonin activity in nine animals.

From these tests, as well as by comparison with known anti-allergy agents of similar type activity (i.e.,Intal) it has been found that the compounds are effective in the treatment of the above mentioned allergic disease states and that such activity is not a function of the classical antihistamine and antiserotonin characteristics. The compounds are effective for their end-use at varying dose ranges, depending on the method of administration. For example, the more active compounds have an effective intraperitoneal administered dose of 0.1-10 MPK, an effective intravenous administered dose of 0.01-10 MPK, and an effective oral dosage of 10-200 MPK. In general, the compounds of this invention, when compared with the clinical effectiveness of Intal-like compounds in aerosol or inhalation preparations are effective at 1-20 mg per day, preferably in divided doses.

In their use as anti-allergy agents, the compounds of this invention are administered in unit dosage forms,

such as tablets, capsules, pills, powders, granules, sterile parenteral solutions or suspensions, eye drops, oral solutions or suspensions, and oil-in-water and water-in-oil emulsions containing suitable quantities of the compounds of this invention (I). The preferred method of administration is by inhalation into the lung or nasal passages by means of an aerosol liquid, or powder for insufflation, or orally.

For oral administration, either solid or fluid unit dosage forms can be prepared. For preparing solid compositions such as tablets, the compounds of this invention (I) are mixed with conventional ingredients such as talc, magnesium stearate, dicalcium phosphate, magnesium aluminum silicate, calcium sulfate, starch, lactose, acacia, methylcellulose, and functionally similar materials as pharmaceutical diluents or carriers. Capsules are prepared by machine encapsulation of a slurry of the compound with an acceptable vegetable oil, light liquid petrolatum or other inert oil.

Fluid unit dosage forms for oral administration such as syrups, elixirs and suspensions can be prepared. The water soluble forms can be dissolved in an aqueous vehicle together with sugar, aromatic flavoring agents and preservatives to form a syrup. An elixier is prepared by using

an alcoholic (ethanol) vehicle with suitable sweeteners such as sugar and saccharin, together with an aromatic flavoring agent.

Suspensions can be prepared with an aqueous vehicle with the aid of a suspending agent such as acacia, tragacanth, methylcellulose and the like.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampul and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the compound is suspended in the vehicle instead of being dissolved and sterilization cannot be accomplished by

filtration. The compound can be sterilized by exposure to ethylene oxide before suspending in the sterile vehicle. Advantageously, a surfactant or wetting agent is included in the composition to facilitate uniform distribution of the compound.

Additionally, a rectal suppository can be employed to deliver the active compound. This dosage form is of particular interest where the mammal cannot be treated conveniently by means of other dosage forms, such as orally or by insufflation, as in the case of young children or debilitated persons. The active compound can be incorporated into any of the known suppository bases by methods known in the art. Examples of such bases include cocoa butter, polyethylene glycols (Carbowaxes), polyethylene sorbitan monostearate, and mixtures of these with other compatible materials to modify the melting point or dissolution rate. These rectal suppositories can weigh from about 1-2.5 g.

For treatment of allergic conditions of the nose, such as rhinitis, compositions adapted for contact with nasal linings are preferred.

Compositions for inhalation are of three basic types: (1) a powder mixture preferably micropulverized with

particle size, preferably from about 1 to about 5 microns; (2) an aqueous solution or suspension to be sprayed with a nebulizer; and (3) an aerosol with volatile propellant in a pressurized container.

The powders are quite simply prepared by mixing a compound of the formula with a solid base which is compatible with lung tissue, preferably lactose. The powders are packaged in a device adapted to emit a measured amount of powder when inhaled through the mouth.

Aqueous solutions are prepared by dissolving the compounds of this invention in water and adding salt to provide an isotonic solution and buffering to a pH compatible with inhalation. The solutions are dispersed in a spray device or nebulizer and sprayed into the mouth while inhaling.

Aerosols are prepared by dispersing a compound of the compounds of this invention (I) in water or ethanol, and mixing with a volatile propellant and placing in a pressurized container having a metering valve to release a predetermined amount of material.

The following are specific examples of effective pharmaceutical formulations by which the compounds of this

invention may be administered.

FORMULATION I

A lot of 10,000 tablets, each containing 1mg of 7-(5'-tetrazolylaminocarbonyl)-(1H,3H,5H)-(1)-benzo-pyrano-(2,3-d)-pyrimidine-4-one-2-thione is prepared from the following types and amounts of ingredients:

| | |
|---|---|
| 7-(5'-tetrazolylaminocarbonyl)-(1H,3H,5H)-(1)-benzopyrano-(2,3-d)-pyrimidine-4-one-2-thione | 500 g |
| Dicalcium phosphate | 1000 g |
| Methylcellulose U.S.P. (15 cps) | 60 g |
| Talc | 150 g |
| Corn starch | 200 g |
| Magnesium stearate | 10 g |

The compound and dicalcium phosphate are mixed well, granulated with 7.5 percent solution of methylcellulose in water, passed through a No.8 screen and dried carefully. The dried granules are passed through a No.12 screen, mixed thoroughly with talc, starch and magnesium stearate, and compressed into tablets.

FORMULATION II

One thousand tablets, each containing 50 mg of 7-(5'-tetrazolylaminocarbonyl-(1H,3H,5H)-(1)-benzopyrano-(2,3-d)-pyrimidine-4-one-2-thione are prepared from the following types and amounts of ingredients:

| | |
|---|---|
| 7-(5'-tetrazolylaminocarbonyl)-(1H,3H,5H)-(1)-benzopyrano-(2,3-d)-pyrimidine-4-one-2-thione | 50 g |
| Microcrystalline cellulose, NF | 410 g |
| Starch | 100 g |
| Magnesium stearate powder | 3 g |

The ingredients are screened and blended together and pressed into tablets.

## FORMULATION III

A sterile preparation suitable for intramuscular injection and containing 5 mg of 7-(5'-tetrazolylamino-carbonyl)-(1H,3H,5H)-(1)-benzopyrano-(2,3-d)-pyrimidine-4-one-2-thione in each milliliter is prepared from the following ingredients:

| | |
|---|---|
| 7-(5'-tetrazolylaminocarbonyl)-(1H,3H,5H)-(1)-benzopyrano-(2,3-d)-pyrimidine-4-one-2-thione | 5 g |
| Benzyl benzoate | 200 ml |
| Methylparaben | 1.5 g |
| Propylparaben | 0.5 g |
| Cottonseed oil q.s. | 1000 ml |

## FORMULATION IV

A powder mixture consisting of 0.1 g of 7-(5'-tetrazolylaminocarbonyl)-(1H,3H,5H)-(1)-benzopyrano-(2,3-d)-pyrimidine-4-one-2-thione and sufficient lactose to make 5 g of mixture is micropulverized and placed in an insufflator designed to deliver 50 mg of powder per dose.

The powder is inhaled into the lungs every 4 to 6 hours for prevention of asthmatic attacks.

The powder is inhaled intranasally every 4 hours for prevention of rhinitis.

## FORMULATION V

Twelve grams of an aerosol composition are prepared from the following ingredients:

| | |
|---|---|
| 7-(5'-tetrazolylaminocarbonyl)-(1H,3H,5H)-(1)-benzopyrano-(2,3-d)-pyrimidine-4-one-2-thione | 0.012 g |
| Freon 12 | 1.440 g |
| Freon 114 | 2.160 g |
| Water | 7.788 g |
| Sorbitan monoleate | 0.600 g |

The compound is dispersed in the water and chilled to -30°C and added to the chilled Freons. The twelve grams of compositions are added to a 13 ml. plastic coated bottle and capped with a metering valve. The metering valve releases 80 mg of composition in an aerosol. The aerosol is inhaled every 4 to 6 hours for prevention of asthmatic attacks.

## FORMULATION VI

The following formulation is suitable for spraying of 0.1 ml per burst of spray from a squeeze bottle.

| | |
|---|---|
| 7-(5'-tetrazolylaminocarbonyl)-(1_H_,3_H_,5_H_)-(1)-benzopyrano-(2,3-d)-pyrimidine-4-one-2-thione NMG* | 10 mg/ml |
| Benzyl alcohol | 10 mg/ml |
| Sorbitol, U.S.P. | 40 mg/ml |
| Water q.s. | 1 ml |

*(calculated on the acid)

As is true in any large class of compounds, it is found that certain sub-groups and certain specific compounds are preferred for the therapeutic utility herein described. In general, it is found that the benzenoid substituent ("R", as defined) ought be

located at the 7- and/or 8-positions, preferably the 7-, that "R" represent carboxamido, or loweralkoxycarbonyl, and that (n) is one. The specifically preferred individual compound is 7-(5'-tetrazolylamino-carbonyl)-(1H,3H,5H)-(1)-benzopyrano(2,3-d)-4-one-2-thione, said compound also being preferably administered as its bis-N-methyl-D-glucamine salt, although other preferred compounds are:

7-(n-propyloxycarbonyl)-(1H,3H,5H)-(1)-benzopyrano-(2,3-d)-4-one-2-thione,

7-(n-butyloxycarbonyl)-(1H,3H,5H)-(1)-benzopyrano-(2,3-d)-4-one-2-thione,

7-(isobutyloxycarbonyl)-(1H,3H,5H)-(1)-benzopyrano-(2,3-d)-4-one-2-thione,

7-(n-propylaminocarbonyl)-(1H,3H,5H)-(1)-benzopyrano-(2,3-d)-4-one-2-thione,

7-(iso-propylaminocarbonyl)-(1H,3H,5H)-(1)-benzopyrano-(2,3-d)-4-one-2-thione,

7-(iso-butylaminocarbonyl)-(1H,3H,5H)-(1)-benzopyrano-(2,3-d)-4-one-2-thione,

7-(2-(2'-pyridyl)-ethylaminocarbonyl)-(1H,3H,5H)-(1)-benzopyrano-(2,3-d)-4-one-2-thione,

7-(2'-hydroxy-2'-phenylethylaminocarbonyl)-(1H,3H,5H)-(1)-benzopyrano-(2,3-d)-4-one-2-thione.

0029934

# CLAIMS

(applicable to all designated states other than Austria)

1. Compounds of the formula

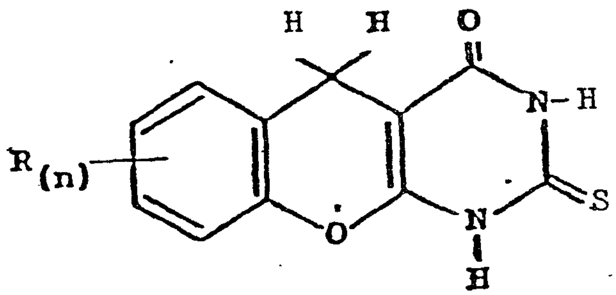

(I)

wherein n is one or two and R represents hydrogen, loweralkyl, lowercycloalkyl, acyloxyloweralkyl, hydroxyloweralkyl, loweralkoxyloweralkyl, nitro, halogeno, hydroxy, loweralkoxy, carboxy, loweralkoxycarbonyl, tetrazolyl, cyano or a carboxamido moiety of the structure

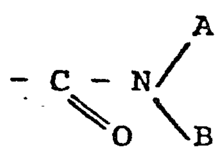

wherein A is straight or branched chain alkyl with up to 12 carbon atoms, lowercycloalkyl, lowercycloalkylloweralkyl, loweralkoxyloweralkyl, hydroxyloweralkyl, fluoroloweralkyl, loweralkenyl, loweralkylthioloweralkyl, loweralkylsulfoxyloweralkyl, loweralkylsulfonylloweralkyl, thiazolyl, oxazolyl, thiadiazolyl, methylthiadiazolyl, furyl, pyrazolyl, tetrazolyl,

methyltetrazolyl, phenyl, or the grouping $-E-R_8$, wherein E is a straight or branched chain or cyclic loweralkylene optionally substituted by hydroxy, alkanoyloxy or phenyl, $R_8$ is phenyl, thiazolyl, oxazolyl, thiadiazolyl, methylthiadiazolyl, tetrazolyl, methyltetrazolyl, furyl, pyridyl, methylpyridyl or piperidinyl; and B is hydrogen, loweralkyl, lowercycloalkyl, lowercycloalkylloweralkyl, or loweralkenyl; or A and B, when taken together with the nitrogen atom to which they are attached, represent imidazolyl, morpholinyl, pyrrolidinyl, piperidinyl or piperazinyl said heterocyclic rings being optionally substituted by hydroxy, loweralkyl or hydroxyloweralkyl; and pharmaceutically acceptable salts thereof.

2. Compounds as claimed in claim 1, characterised by one or both of the following features:

a) R is located in positions 7 and/or 8; and

b) R is selected from halogeno, carboxy, loweralkoxycarbonyl, acyloxyloweralkyl and the carboxamido moiety as defined in claim 1.

3. Compounds as claimed in claim 1 or 2 wherein, in the carboxamido moiety of the structure as defined in claim 1, A is tetrazolyl or the grouping $-E-R_8$, wherein E is straight chain alkylene with

up to three carbon atoms and is optionally substituted by hydroxy, and $R_8$ is phenyl, tetrazolyl or pyridyl; and B is hydrogen or loweralkyl, or A and B, when taken together with the nitrogen atom to which they are attached, represent N-imidazolyl; the carboxamido moiety being in position 7.

4. A compound of any one of claims 1 to 3, namely 7-(2-[2-pyridyl]ethylaminocarbonyl)-(1H,3H,5H)-(1)-benzopyrano-(2,3-d)-pyrimidine-4- one -2-thione, 7-(N-imidazolylcarbonyl)-(1H,3H,5H)-(1)-benzopyrano-(2,3-d)-pyrimidine-4-one-2-thione or 7-(5-tetrazolylaminocarbonyl)-(1H,3H,5H)-(1)-benzopyrano-(2,3-d)-pyrimidine-4-one-2-thione.

5. A compound of any one of claims 1 to 4, namely the bis-N-methyl-D-glucamine salt of 7-(5-tetrazolylaminocarbonyl)-(1H,3H,5H)-(1)-benzopyrano-(2,3-d)-pyrimidine-4-one -2-thione.

6. Pharmaceutical compositions containing as active ingredient at least one compound as claimed in any one of claims 1 to 5 together with a pharmaceutical carrier or excipient.

7. Process for the preparation of a compound of the formula (I) as defined in claim 1, or of a pharmaceutically acceptable salt thereof; characterised in that one of steps A) to D) is applied:

A) reducing a compound of the formula

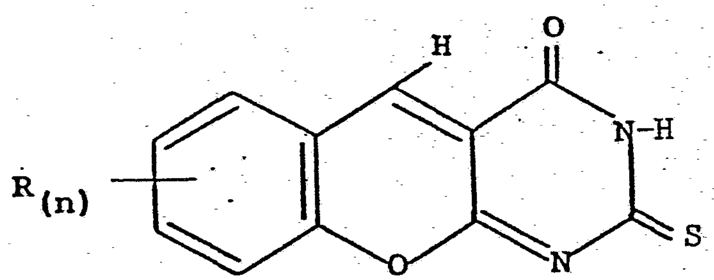

(IV)

wherein R and n are as defined in claim 1; or

B) for the preparation of compounds of formula I, wherein R is not acyloxyloweralkyl; cyclising a compound of the formula

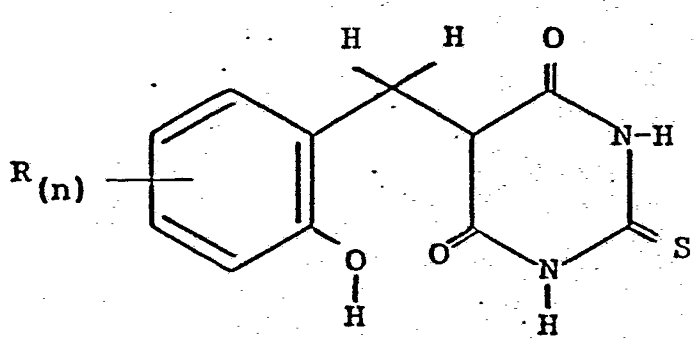

(VII)

wherein R and n are as defined in claim 1 with the proviso that R is not acyloxyloweralkyl; and wherein the hydroxy group in position 2 of the phenyl moiety may be protected; or

C) reacting a compound of the formula

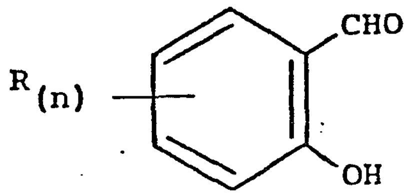

(II)

wherein R and n are as defined in claim 1, with 2-thiobarbituric acid in an alcoholic solvent and in the presence of a sulfonic acid; or

D) for the preparation of compounds of formula I, wherein R and n are as defined in claim 1, with the same proviso for substituent R as in process step B); reacting a compound of the formula

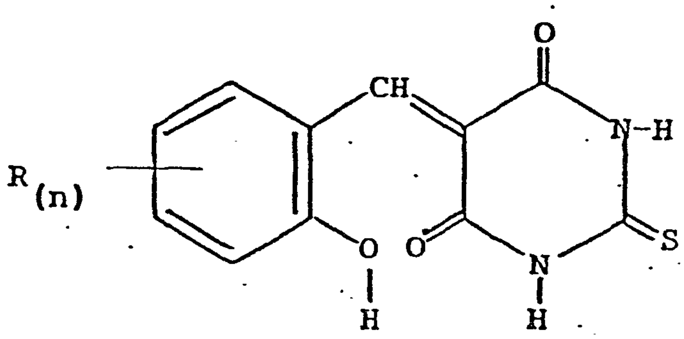

(VI)

in an alcoholic solvent and in the presence of a sulfonic acid;

any of these steps A) to D) being followed by one or more of steps (i) to (xiii) to obtain a compound of formula I or to convert a compound of formula I into another compound of formula I:

(i) esterification of the carboxyl group representing substituent R;

(ii) de-esterification of an alkoxycarbonyl group;

(iii) trans-esterification of an alkoxycarbonyl group;

(iv) acylation of any hydroxy group in substituent R or E;

(v) de-acylation of acyloxyloweralkyl;

(vi) de-etherification of alkoxyloweralkyl;

(vii) etherification of any hydroxyl group in/or representing substituent R;

(viii) hydrolysing or alcoholysing the cyano group representing substituent R;

(ix) oxidation of a hydroxymethyl group representing substituent R;

(x) amidation of the carboxyl group representing substituent R to obtain the carboxamido moiety as defined above;

(xi) trans-amidation of a carboxamido moiety representing substituent R;

(xii) de-amidation of a carboxamido moiety representing substituent R;

(xiii) preparing a pharmaceutically acceptable salt of any of the compounds so obtained.

8. Process for the preparation of pharmaceutical compositions characterised in that a compound of formula I as defined in claim 1 or a pharmaceutically acceptable salt thereof is admixed with a pharmaceutical carrier or excipient.

9. A compound as claimed in claim 1, whenever obtained by a process as claimed in claim 7.

CLAIMS

(applicable to Austria )

1. Process for the preparation of a compound of the formula

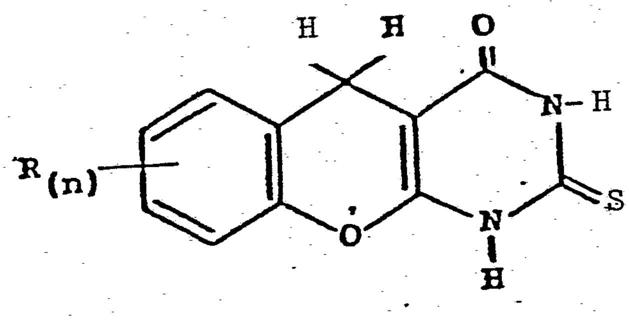

(I)

wherein n is one or two and R represents hydrogen, loweralkyl, lowercycloalkyl, acyloxyloweralkyl, hydroxyloweralkyl, loweralkoxyloweralkyl, nitro, halogeno, hydroxy, loweralkoxy, carboxy, loweralkoxycarbonyl, tetrazolyl, cyano or a carboxamido moiety of the structure

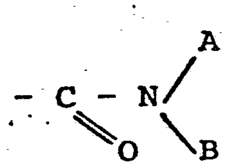

wherein A is straight or branched chain alkyl with up to 12 carbon atoms, lowercycloalkyl, lowercycloalkylloweralkyl, loweralkoxyloweralkyl, hydroxyloweralkyl, fluoroloweralkyl, loweralkenyl, loweralkylthioloweralkyl, loweralkylsulfoxyloweralkyl, loweralkylsulfonylloweralkyl, thiazolyl, oxazolyl, thiadiazolyl, methylthiadiazolyl, furyl, pyrazolyl, tetrazolyl, methyltetrazolyl, phenyl or the grouping $-E-R_8$, wherein E is a straight or branched chain or cyclic loweralkylene optionally substituted by hydroxy, alkanoyloxy or phenyl, $R_8$ is phenyl, thiazolyl, oxazolyl, thiadiazolyl, methylthiadiazolyl, tetrazolyl, methyltetrazolyl, furyl, pyridyl, methylpyridyl or piperidinyl; and B is hydrogen, loweralkyl, lowercycloalkyl, lowercycloalkylloweralkyl, or loweralkenyl; or A and B, when taken together with the nitrogen atom to which they are attached, represent imidazolyl, morpholinyl, pyrrolidinyl, piperidinyl or piperazinyl said heterocyclic rings being optionally substituted by hydroxy, loweralkyl or hydroxyloweralkyl; or of a pharmaceutically acceptable salt thereof; characterised in that one of steps A) to D) is applied:

A) reducing a compound of the formula

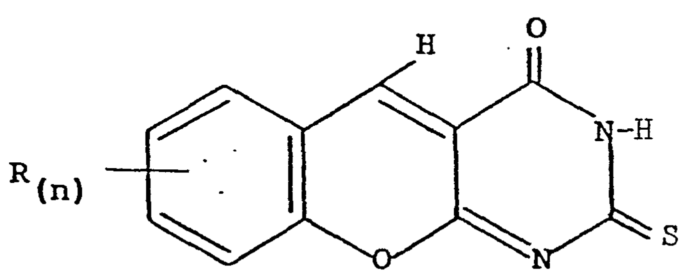

(IV)

wherein R and n are as defined above; or

B) for the preparation of compounds of formula I, wherein R is not acyloxyloweralkyl; cyclising a

compound of the formula

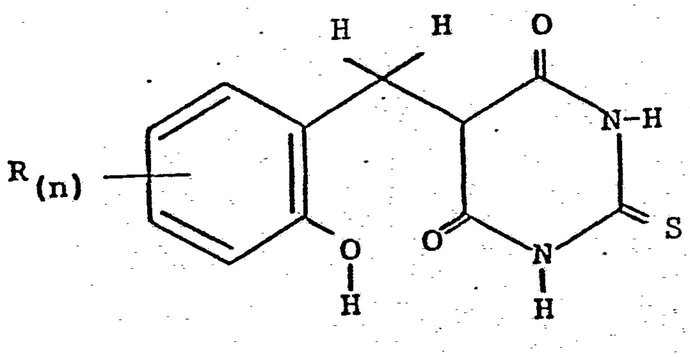

(VII)

wherein R and n are as defined above with the proviso that R is not acyloxyloweralkyl; and wherein the hydroxy group in position 2 of the phenyl moiety may be protected; or

C) reacting a compound of the formula

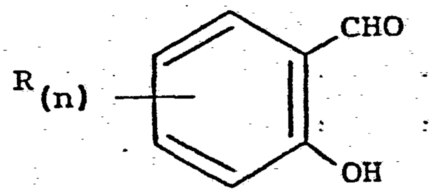

(II)

wherein R and n are as defined above, with 2-thiobarbituric acid in an alcoholic solvent and in the presence of a sulfonic acid; or

D) for the preparation of compounds of formula I, wherein R and n are as defined above, with the same proviso for substituent R as in process step B); reacting a compound of the formula

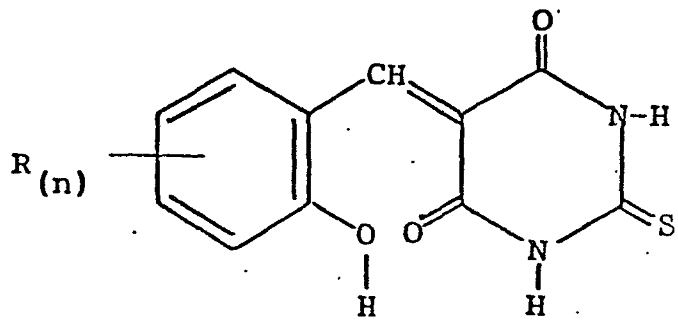

(VI)

in an alcoholic solvent and in the presence of a sulfonic acid;

any of these steps A) to D) being followed by one or more of steps (i) to (xiii) to obtain a compound of formula I or to convert a compound of formula I into another compound of formula I:

(i) esterification of the carboxyl group representing substituent R;

(ii) de-esterification of an alkoxycarbonyl group;

(iii) trans-esterification of an alkoxycarbonyl group;

(iv) acylation of any hydroxy group in substituent R or E;

(v) de-acylation of acyloxyloweralkyl;

(vi) de-etherification of alkoxyloweralkyl;

(vii) etherification of any hydroxyl group in/or representing substituent R;

(viii) hydrolysing or alkoholysing the cyano group representing substituent R;

(ix) oxydation of a hydroxymethyl group representing substituent R;

(x) amidation of the carboxyl group representing substituent R to obtain the carboxamido moiety as defined above;

(xi) trans-amidation of a carboxamido moiety representing substituent R;

(xii) de-amidation of a carboxamido moiety representing substituent R;

(xiii) preparing a pharmaceutically acceptable salt of any of the compounds so obtained.

2. Process as claimed in claim 1, characterised in that 7-(5-tetrazolylaminocarbonyl)-(1H,3H,5H)-(1)-benzopyrano-(2,3-d)-pyrimidine-4-one-2-thione or a pharmaceutically acceptable salt thereof is prepared.

3. Process as claimed in claim 1 or 2, characterised in that in step A) reduction is effected with sodium borohydride or sodium cyanoborohydride in an alcoholic medium.

4. Process as claimed in claim 1 or claim 2, characterised in that in step C) and D) the reaction is carried out in n-propanol, n-butanol or iso-propanol in the presence of methanesulfonic acid or p-toluenesulfonic acid.

5. A compound of formula I as defined in claim 1 or a pharmaceutically acceptable salt thereof, whenever obtained by a process as claimed in any one of claims 1 to 4.

6. Process for the preparation of pharmaceutical compositions characterised in that a compound of formula I as defined in claim 1 or a pharmaceutically acceptable salt thereof is admixed with a pharmaceutical carrier or excipient.

7. Process for the preparation of pharmaceutical compositions characterised in that a compound of formula I or a pharmaceutically acceptable salt thereof, whenever obtained by the process as claimed in any one of claims 1 to 4, is admixed with a pharmaceutical carrier or excipient.

0029934

European Patent Office

# EUROPEAN SEARCH REPORT

Application number

EP 80106869.3

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| | US - A - 4 128 648 (ROVNYAK)<br>+ Columns 1,2,3 +<br>-- | 1,6,8 | C 07 D 491/052<br>A 61 K 31/505//<br>(C 07 D 491/052<br>C 07 D 239/00<br>C 07 D 311/00) |
| | US - A - 3 896 128 (KAST et al.)<br>+ Columns 1,2 +<br>---- | 1,7 | |

TECHNICAL FIELDS SEARCHED (Int.Cl. 3)

C 07 D 491/00
C 07 D 513/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 23-01-1981 | LUX |